# DEMANDE DE BREVET EUROPEEN

(11) **EP 3 960 686 A1**
(43) Date de publication de la demande: **02.03.2022**
(21) Numéro de dépôt: 21194079.6
(22) Date de dépôt: 31.08.2021
(51) Int. Cl.: B66B 29/00, G07C 9/25

(54) **INSTALLATION DE CONTRÔLE D ACCÈS**

(30) Priorité: 01.09.2020 FR 2008883
(71) Demandeur: Idemia Identity & Security France, 92400 Courbevoie (FR)
(72) Inventeur: TOURET, Olivier, 92400 COURBEVOIE (FR); CHASTEL, Pierre, 92400 COURBEVOIE (FR)
(74) Mandataire: Lavialle, Bruno François Stéphane

(57) **Abrégé**

Installation de contrôle d'accès, comprenant un bâti (1), un trottoir roulant (7) qui s'étend entre deux murs (2) du bâti depuis une entrée (3.1) jusqu'à une sortie (3.2) de l'installation, un lecteur d'un document (10) monté sur le bâti (1) au voisinage de l'entrée (3.1) de l'installation, un dispositif de capture biométrique (11, 12) monté sur le bâti (1) à une distance prédéterminée (d2) de la sortie (3.2) de l'installation, et une unité électronique de traitement (13) reliée au lecteur de document (10) et au dispositif de capture biométrique (11, 12) et agencée pour comparer des données biométriques de référence contenues dans le document et des caractéristiques biométriques fournies par le dispositif de capture biométrique (11, 12) pour en vérifier la correspondance, la distance prédéterminée étant suffisante pour que l'unité électronique de traitement (13) réalise l'opération de vérification avant que l'usager concerné atteigne la sortie (3.2) de l'installation compte tenu d'une vitesse du trottoir roulant (7).

## Description

La présente invention concerne le domaine de l'identification et la reconnaissance des individus par exemple pour pénétrer dans une zone d'accès protégé, comme une salle d'embarquement d'un aéroport, ou le passage d'une frontière entre deux états.

### ARRIERE PLAN DE L'INVENTION

Classiquement, les contrôles d'identité permettant d'accéder à de telles zones sont réalisés par des opérateurs. Le temps d'accès est alors relativement long, en particulier en période de forte affluence comme cela peut se produire au moment des départs en vacances dans les aéroports.

Il a été imaginé un dispositif automatique comportant:
- un sas;
- un lecteur de circuit intégré à l'entrée du sas pour lire des données biométriques de référence mémorisées dans une mémoire incorporée à un passeport; et
- un dispositif de capture biométrique disposé à l'intérieur du sas pour capturer une image d'une partie du corps du titulaire du passeport et en extraire des données biométriques à comparer aux données biométriques de référence.

Cependant, un tel dispositif permet certes de se passer d'un opérateur mais il reste nécessaire de multiplier les dispositifs pour les lieux de forte affluence.

### OBJET DE L'INVENTION

L'invention a notamment pour but d'améliorer les vérifications de documents, notamment d'identité ou des autorisations d'accès, et/ou les vérifications biométriques dans les zones de relativement forte affluence.

### RESUME DE L'INVENTION

A cet effet, on prévoit, selon l'invention une installation de contrôle d'accès, comprenant un bâti, une surface qui s'étend entre deux murs du bâti et qui est mobile par rapport au bâti et parallèlement aux murs pour former un sol sur lequel des usagers sont destinés à se tenir debout, une motorisation pour entraîner en translation la surface mobile entre les murs depuis une entrée de l'installation jusqu'à une sortie de l'installation. L'installation comprend également au moins l'un d'un lecteur d'un document et d'un dispositif de capture biométrique, monté sur le bâti au à une distance prédéterminée de la sortie de l'installation, et une unité électronique de traitement reliée au lecteur de document et/ou au dispositif de capture biométrique et agencée pour comparer des données de référence aux données fournies par le lecteur de document et/ou le dispositif de capture biométrique pour en vérifier la correspondance. La distance prédéterminée est suffisante pour que l'unité électronique de traitement réalise l'opération de vérification avant que l'usager concerné atteigne la sortie de l'installation compte tenu d'une vitesse de déplacement de la surface mobile.

Ainsi, l'opération de vérification est réalisée alors que les usagers sont en mouvement, ce qui permet d'effectuer les vérifications en temps masqué, pendant le cheminement des usagers vers leur destination comme une salle d'embarquement d'aéroport ou un passage de frontière notamment.

Selon une caractéristique particulière de l'invention, le dispositif de capture biométrique comprend une caméra optique pour capturer des images du visage des usagers et l'unité électronique de traitement est agencée pour détecter, à partir des images, au moins l'un des paramètres physiologiques suivant:
- une température des usagers;
- une brillance des yeux des usagers;
- une dilatation des pupilles des usagers;
- une ouverture des yeux;
- une sudation;
- un rythme cardiaque des usagers.

Ainsi, il est possible d'utiliser les images de la caméra à la fois à des fins biométriques mais aussi pour déterminer un état physiologique du candidat, les signes en question pouvant être des symptomes d'une anxiété, d'une maladie, de la prise de substances...

Avantageusement, l'unité électronique de traitement est agencée pour évaluer à partir du ou des paramètres physiologiques un état de santé des usagers et/ou est agencée pour détecter la présence ou l'absence d'un masque sur le visage des usagers.

De préférence, l'installation comprend un lecteur d'un document et un dispositif de lecture biométrique, le lecteur de document étant monté sur le bâti au voisinage de l'entrée de l'installation et le dispositif de capture biométrique étant monté sur le bâti à la distance prédéterminée de la sortie de l'installation, et l'unité électronique de traitement est reliée au lecteur de document et au dispositif de capture biométrique et est agencée pour comparer des données biométriques de référence et les caractéristiques biométriques fournies par le dispositif de capture biométrique pour en vérifier la correspondance.

D'autres caractéristiques et avantages de l'invention ressortiront à la lecture de la description qui suit d'un mode de réalisation particulier et non limitatif de l'invention.

### BREVE DESCRIPTION DES DESSINS

Il sera fait référence aux dessins annexés, parmi lesquels:
- La figure 1 est une vue schématique en perspective d'une installation selon l'invention;
- La figure 2 est une vue schématique en bout et en coupe partielle de cette installation;
- La figure 3 est une vue schématique de cette installation en coupe longitudinale selon le plan III de la figure 1;
- La figure 4 est une vue schématique partielle de l'extrémité d'un des murs à la sortie de l'installation;
- La figure 5 est une vue schématique partielle de dessus d'une des mains courantes;
- La figure 6 est une vue schématique partielle de cette main courante selon une variante de réalisation, en coupe transversale selon la ligne VI-VI de la figure 5.

### DESCRIPTION DETAILLEE DE L'INVENTION

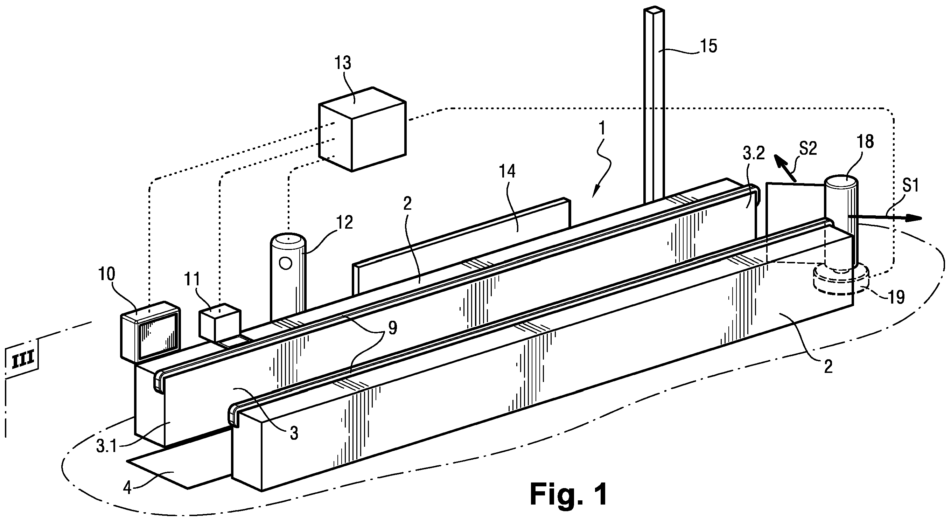

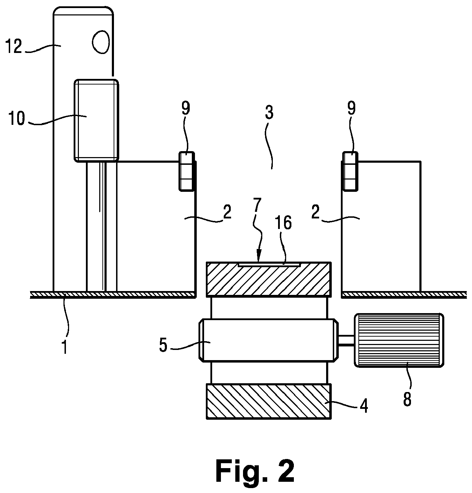

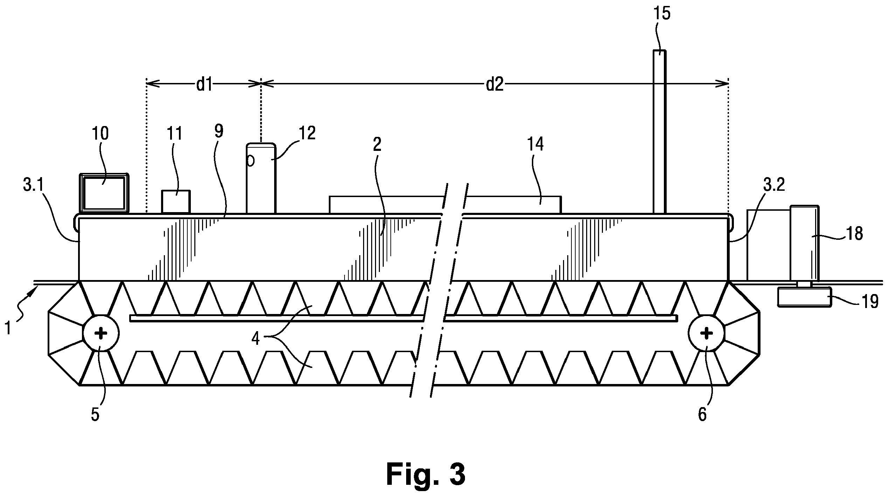

En référence aux figures 1 à 5, l'installation de contrôle d'accès selon l'invention comprend un bâti généralement désigné en 1 comportant deux murs 2 délimitant entre eux un couloir 3 ayant une entrée 3.1 et une sortie 3.2.

Dans le couloir 3, le long des deux murs 2, s'étend un trottoir roulant 4 qui est mobile par rapport au bâti 1 et parallèlement aux murs 2. Le trottoir roulant 4 est une chaîne ayant des maillons articulés les uns aux autres et entourant deux poulies 5, 6 en formant une boucle allongée ayant un brin supérieur et un brin inférieur. Les maillons, lorsqu'ils sont dans le brin supérieur de la boucle, ont une surface supérieure formant un sol 7 sur lequel des usagers sont destinés à se tenir debout. La poulie 6 est entrainée en rotation par un moteur 8 pour déplacer chaque maillon du brin supérieur depuis l'entrée 3.1 vers la sortie 3.2 (et les maillons du brin inférieur dans le sens inverse bien évidemment). Le trottoir roulant 4 peut être réalisé différemment par exemple au moyen d'un tapis roulant.

Une main courante 9 est monté sur chaque mur 2. Chaque main courante 9 forme une boucle allongée autour de poulies non visibles en ayant un brin supérieur s'étendant sur la surface supérieure du mur 2 et un brin inférieur courant dans le mur 2. L'une des poulies est motorisée pour entraîner la main courante 9 en synchronisation avec le trottoir roulant 4.

La motorisation de la main courante 9 et du trottoir roulant 4 est programmée pour autoriser une vitesse de déplacement V des usagers de l'installation. La vitesse V est par exemple comprise entre 0,5 et 1 m.s⁻¹.

L'installation comprend en outre un lecteur 10 de document sans contact, un capteur d'empreinte 11 et une caméra de capture de visage 12 qui sont tous trois reliés à une unité électronique de traitement 13.

Le lecteur 10 est agencé pour lire une mémoire incorporée à un document. La mémoire est ici un circuit intégré relié à une antenne. Le lecteur 10 est connu en lui-même et comprend un processeur relié à une antenne et programmé pour interroger le circuit intégré et obtenir les données qu'il contient. En l'occurrence, le document est un passeport et le circuit intégré contient des données biométriques du titulaire du passeport, ici des données biométriques d'empreinte digitale et des données biométriques de visage. Le lecteur 10 est monté sur l'un des murs 2 au voisinage de l'entrée 3.1 du couloir 3.

Le capteur d'empreinte 11 est ici un capteur optique ayant un champ couvrant une zone de capture dans laquelle l'usager doit passer la main pour permettre au capteur de saisir au moins une image des dermatoglyphes d'un ou plusieurs doigts de l'usager. Le capteur a une vitesse d'acquisition et une résolution permettant l'acquisition des images des mains en mouvement avec un détail suffisant pour autoriser l'extraction de caractéristiques biométriques d'empreinte. Le capteur d'empreinte 11 est monté sur l'un des murs 2 au voisinage de l'entrée 3.1 du couloir 3.

La caméra de capture de visage 12 comprend un capteur ayant une vitesse d'acquisition et une résolution permettant l'acquisition des images des visages en mouvement avec un détail suffisant pour autoriser l'extraction de caractéristiques biométriques de visage. La caméra de capture 12 est ici montée dans un poteau ayant une base fixée au bâti 1 à côté du mur 2. La caméra de capture de visage 12 a un champ couvrant une profondeur d1 le long du couloir 3, distance suffisante pour que la caméra de capture de visage 12 puisse prendre au moins une image du visage de l'usager compte tenu de la vitesse de déplacement V de l'usager dans le couloir 3.

L'unité électronique de traitement 13 comprend un processeur et des mémoires contenant des programmes de reconnaissance biométrique agencés pour comparer, d'une part, les données biométriques de référence contenues dans les documents et, d'autre part, les caractéristiques biométriques fournies par le capteur d'empreinte 11 et la caméra de capture 12, pour en vérifier la correspondance. Ces programmes de reconnaissance biométrique, couramment appelés programmes de matching, sont connus en eux-mêmes.

Le capteur d'empreinte 11 et la caméra de capture de visage 12 sont placés le long du couloir 3 à une distance d2 de la sortie 3.2 du couloir 3. Cette distance d2 est suffisante pour que l'unité électronique de traitement 13 réalise l'opération de vérification biométrique avant que l'usager concerné atteigne la sortie 3.2 du couloir 3 compte tenu de la vitesse de déplacement V de la surface mobile.

L'installation comprend ici également une barrière 18 disposée à l'extérieur du couloir 3 au voisinage de la sortie 3.2 de l'installation et reliée à un moteur 19 pour être mobile entre deux positions pour diriger les usagers soit dans une direction S1 soit dans une direction S2. Le moteur 19 est relié à l'unité de traitement 13 qui commande le déplacement de la barrière vers l'une ou l'autre position en fonction du résultat de la vérification biométrique. Par exemple, lorsque la vérification biométrique est positive, l'usager est amené par la direction S1 vers une sortie directe sans contrôle supplémentaire alors que, en cas de vérification biométrique négative, l'usager est amené par la direction S2 vers un opérateur chargé d'effectuer des contrôles supplémentaires.

De préférence, les usagers seront séparés d'une cinquantaine de centimètres pour faciliter les opérations de capture et bien discriminer les usagers les uns des autres.

Avantageusement, l'unité électronique de traitement 13 exécute également un programme permettant de vérifier la compatibilité des caractéristiques biométriques avec des informations personnelles de l'usager figurant dans la mémoire du passeport, comme l'âge, le genre, la couleur des yeux...

De préférence, l'unité électronique de traitement 13 est agencée pour détecter, à partir des images fournies par la caméra de capture 12, au moins l'un des paramètres physiologiques suivant:
- une température des usagers;
- une brillance des yeux des usagers;
- une dilatation des pupilles des usagers;
- une ouverture des yeux;
- une sudation;
- un rythme cardiaque des usagers.

L'unité électronique de traitement 13 est agencée pour évaluer à partir du ou des paramètres physiologiques un état de santé des usagers. De préférence, l'unité électronique met en oeuvre un réseau de neurones pour classifier les usagers en usagers présumés en bonne santé et usagers présumés en mauvaise santé à partir des paramètres physiologiques. On prévoira avantageusement la prise en compte de la température ambiante lors de cette évaluation.

L'unité électronique de traitement 13 est également agencée pour détecter la présence d'un masque de protection (type masque chirurgical couvrant la bouche et le nez) sur le visage des usagers. L'algorithme de reconnaissance faciale sera de préférence configuré pour autoriser une reconnaissance biométrique malgré la présence d'un tel masque de protection (en privilégiant les caractéristiques biométriques laissées découvertes par le masque).

L'installation comprend en outre des capteurs additionnels, à savoir:
▪ un capteur d'unicité comportant une caméra, une ligne d'éclairage horizontale 14 et une ligne d'éclairage verticale 15 pour pouvoir distinguer l'un de l'autre deux usagers qui seraient collés l'un à l'autre (l'un derrière l'autre, l'un sur l'autre, l'un à côté de l'autre...). On notera que plus le temps de parcours du couloir 3 est long, plus il sera difficile à deux usagers de rester collés sans que cela soit détecté;
• un capteur de poids 17 monté sur chaque maillon formant le trottoir roulant 4. La mesure du poids peut permettre de révéler que deux usagers se trouvent sur le même maillon, collés l'un à l'autre.

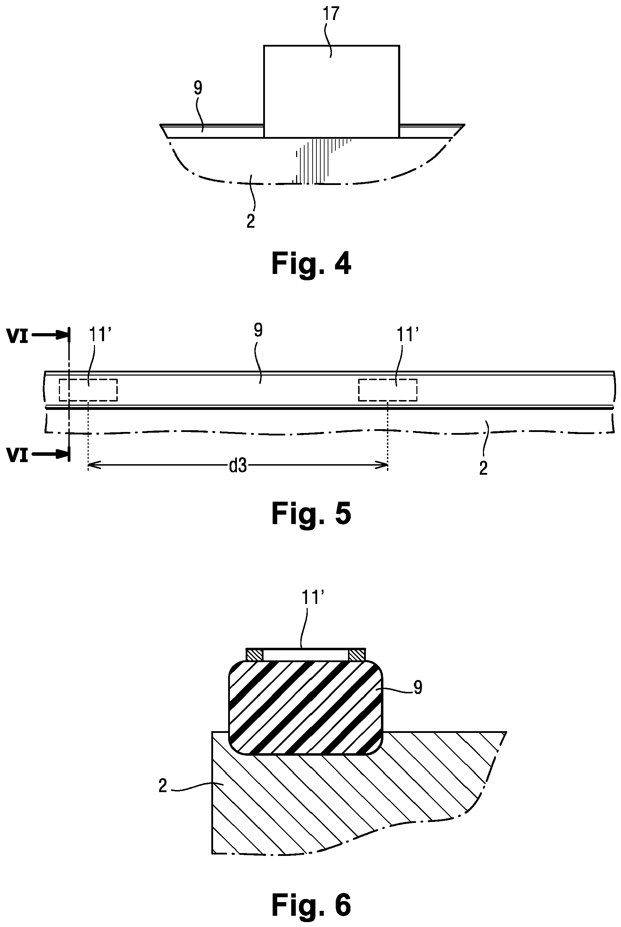

Comme visible à la figure 5, l'installation comprend un tunnel de désinfection 17 monté sur chaque mur 2 et dans lequel défile la main courante 9. Le tunnel de désinfection 17 comprend par exemple une lampe UV pour projet un rayonnement ultraviolet vers la surface supérieure de la main courante 9 et un pulvérisateur d'une solution désinfectante en direction de la surface supérieure de la main courante 9.

En variante, des capteurs d'empreinte 11' peuvent être placés directement sur une des mains courantes. Les capteurs d'empreinte 11' sont des capteurs de type TFT ou couches minces ou capacitif. L'emplacement des capteurs d'empreinte 11' est rendu visible sur la surface supérieure de la main courante 9 pour que les usagers sachent où ils doivent poser leur main pour permettre la capture des empreintes. Les capteurs d'empreinte 11' sont espacés les uns des autres d'une distance d3 correspondant à l'écartement souhaité des usagers sur le trottoir roulant 4.

Bien entendu, l'invention n'est pas limitée au mode de réalisation décrit mais englobe toute variante entrant dans le champ de l'invention telle que définie par les revendications.

En particulier, l'installation peut avoir une structure différente de celle décrite et par exemple prendre la forme d'un trottoir roulant incliné ou d'un escalier mécanique.

La mémoire incorporée au document peut avoir la forme d'un circuit intégré, d'une bande magnétique, d'un code-barres, d'une image... Le lecteur de document peut être une caméra optique dans le champ de laquelle doit être présenté le code-barres ou l'image.

L'installation peut comprendre au moins un des dispositifs de capture biométrique suivant:
- un capteur d'empreinte digitale;
- un capteur d'iris;
- un capteur de visage.

L'installation peut comprendre, ou pas, au moins un capteur additionnel relié à l'unité de traitement, parmi:
- un capteur de poids disposé sur la surface mobile;
- un capteur de hauteur des usagers;
- une caméra à onde millimétrique ou caméra terahertz;
- un capteur de température des usagers ;
- un détecteur d'explosif;
- un détecteur d'unicité en prévoyant des éclairages horizontaux et verticaux le long du tapis;
- un lecteur de ticket d'embarquement à l'entrée de l'installation...

L'invention est utilisable avec des passeports dont la mémoire ne comprend que des données biométriques de visage. Il reste intéressant, même si ce n'est pas obligatoire, d'effectuer malgré tout une capture d'empreintes digitales afin par exemple d'effectuer, à partir de celles-ci, des recherches dans des bases de données de personnes recherchées ou de personnes disparues, vérifier des visas... Ceci peut également s'appliquer à d'autres types de biométrie.

Pour la position des capteurs biométriques, la seule contrainte est qu'ils soient situés à une distance de la sortie qui permette de faire les contrôles ad hoc avant la sortie.

Il peut y avoir plusieurs caméras de capture (par exemple pour améliorer la précision) notamment à droite et à gauche de la surface mobile.

Les capteurs biométriques permettant de capturer des biométries à distance et en mouvement sont particulièrement adaptées à l'invention (par exemple les capteurs de visage et les capteurs d'iris).

Le lecteur de document peut comprendre un organe de lecture optique pour réaliser une phase de lecture optique du document et des organes répartis dans ou le long de la main courante pour assurer une lecture sans contact du circuit intégré du document. La lecture de la puce peut aussi se faire en parallèle de l'acquisition de la biométrie.

Les données biométriques de référence peuvent être contenues dans le document d'identité ou dans une base de données hébergée sur un serveur auquel le dispositif est connecté. La recherche dans la base de données est de préférence réalisée à partir d'informations contenues dans le document (numéro du document, nom du titulaire...).

L'installation peut ne pas comprendre de lecteur de document. Dans tous les cas, le ou les lecteurs de document et le ou les dispositifs de capture biométrique sont positionnés le long du bâti à une distance suffisante pour que l'unité électronique de traitement 13 réalise l'opération de vérification avant que l'usager concerné atteigne la sortie 3.2 de l'installation compte tenu de la vitesse de déplacement V de la surface mobile 7.

On peut prévoir que l'unité de traitement 13 puisse piloter le moteur 8 pour ralentir le trottoir roulant 4 lorsque la vérification biométrique d'un usager prend plus de temps que prévu de telle manière que cette vérification biométrique soit terminée lorsque l'usager atteint la sortie du couloir 3.

La barrière est facultative. L'aiguillage peut par exemple se faire par un opérateur ou une signalétique.

## Revendications

1. Installation de contrôle d'accès, comprenant un bâti (1), une surface (7) qui s'étend entre deux murs (2) du bâti (1) et qui est mobile par rapport au bâti (1) et parallèlement aux murs (2) pour former un sol sur lequel des usagers sont destinés à se tenir debout, une motorisation (8) pour entraîner en translation la surface mobile (7) entre les murs (2) depuis une entrée (3.1) de l'installation jusqu'à une sortie (3.2) de l'installation; et au moins l'un, d'un lecteur d'un document (10) et d'un dispositif de capture biométrique, monté sur le bâti (1) à une distance prédéterminée (d2) de la sortie (3.2) de l'installation, et une unité électronique de traitement (13) reliée au lecteur de document (10) et/ou au dispositif de capture biométrique (11, 12) et agencée pour comparer des données de référence aux données fournies par le lecteur de document (10) et/ou le dispositif de capture biométrique (11, 12) pour en vérifier la correspondance, la distance prédéterminée étant suffisante pour que l'unité électronique de traitement (13) réalise l'opération de vérification avant que l'usager concerné atteigne la sortie (3.2) de l'installation compte tenu d'une vitesse de déplacement (V) de la surface mobile (7).

2. Installation selon la revendication 1, dans laquelle le dispositif de capture biométrique comprenant une caméra optique (12) pour capturer des images du visage des usagers.

3. Installation selon la revendication 2, dans laquelle l'unité électronique de traitement (13) est agencée pour détecter, à partir des images, au moins l'un des paramètres physiologiques suivant:
• une température des usagers;
• une brillance des yeux des usagers;
• une dilatation des pupilles des usagers;
• une ouverture des yeux;
• une sudation;
• un rythme cardiaque des usagers.

4. Installation selon la revendication 3, dans laquelle l'unité électronique de traitement (13) est agencée pour évaluer à partir du ou des paramètres physiologiques un état de santé des usagers.

5. Installation selon l'une quelconque des revendications 2 à 4, dans laquelle l'unité électronique de traitement (13) est agencée pour détecter la présence ou l'absence d'un masque sur le visage des usagers.

6. Installation selon l'une quelconque des revendications précédentes, comprenant un lecteur d'un document (10) et un dispositif de capture biométrique, le lecteur de document étant monté sur le bâti 1 au voisinage de l'entrée (3.1) de l'installation et le dispositif de capture biométrique (11, 12) étant monté sur le bâti (1) à la distance prédéterminée (d2) de la sortie (3.2) de l'installation, et l'unité électronique de traitement (13) est reliée au lecteur de document (10) et au dispositif de capture biométrique (11, 12) et est agencée pour comparer des données biométriques de référence et les caractéristiques biométriques fournies par le dispositif de capture biométrique (11, 12) pour en vérifier la correspondance.

7. Installation selon l'une quelconque des revendications précédentes, comprenant au moins un des dispositifs de capture biométrique suivant:
• un capteur d'empreinte digitale (11, 11');
• un capteur d'iris;
• un capteur de visage (12).

8. Installation selon l'une quelconque des revendications précédentes, comprenant au moins un capteur additionnel relié à l'unité de traitement (13), parmi:
▪ un capteur de poids (16) disposé sur la surface mobile;
▪ un capteur de hauteur des usagers;
▪ une caméra à onde millimétrique ou caméra terahertz;
▪ un capteur de température des usagers.

9. Installation selon l'une quelconque des revendications précédentes, comprenant un détecteur d'unicité (14, 15) permettant de discriminer deux usagers collés l'un à l'autre.

10. Installation selon l'une quelconque des revendications précédentes, comprenant des mains courantes (9) courant sur la surface supérieure des murs (2) et se déplaçant de manière synchronisée avec la surface mobile (7).

11. Installation selon la revendication 10, comprenant un organe (17) de désinfection de chaque main courante (9).

12. Installation selon la revendication 10 ou la revendication 11, comprenant plusieurs dispositifs de capture biométrique comportant un capteur d'empreinte (11'), les capteurs d'empreinte (11') étant fixés sur une des mains courantes (9) de manière espacée les uns des autres.

13. Installation selon l'une quelconque des revendications précédentes, comprenant une barrière (18) disposée à la sortie (3.2) de l'installation et reliée à un moteur (19) commandé par l'unité de traitement pour amener la barrière (18) dans deux positions distinctes en fonction du résultat de la vérification.

14. Installation selon l'une quelconque des revendications précédentes, dans laquelle l'unité électronique de traitement (13) est agencée pour vérifier la compatibilité des caractéristiques biométriques extraites avec au moins une information relative à l'usager figurant sur le document.
